(19) Europäisches Patentamt

European Patent Office

Office européen des brevets

(11) Publication number: **0 465 921 A1**

## (12) EUROPEAN PATENT APPLICATION

(21) Application number: **91110494.1**

(22) Date of filing: **25.06.91**

(51) Int. Cl.5: **A23G 3/30**, A61K 9/68,
A61K 7/22, A23G 3/30 +

(30) Priority: **26.06.90 IT 2076690**

(43) Date of publication of application:
**15.01.92 Bulletin 92/03**

(84) Designated Contracting States:
**DE FR GB**

(71) Applicant: **ISCOFAR SAS DI PAOLO E.
GHIRARDI**
**Viale Bianca Maria, 10**
**I-20129 Milano(IT)**

(72) Inventor: **Ouarto Dipalo Chiara, C/O Iscofar
SAS**
**Viale Bianca Maria, 10**
**20129 Milano(IT)**
Inventor: **Ghirardi, Paolo E., C/O Iscofar SAS**
**Viale Bianca Maria, 10**
**20129 Milano(IT)**

(74) Representative: **Dragotti, Gianfranco**
**SAIC BREVETTI s.r.l. Viale Bianca Maria, 15**
**I-20122 Milano(IT)**

(54) Chewing gum composition for the prevention and treatment of dental plaque.

(57) By incorporating N-acetylcysteine in a chewing gum composition the dental plaque is strongly reduced and consequently the dental caries is prevented.

The present invention relates to a composition for the prevention and treatment of the dental plaque.

The diseases of the oral cavity are classified among the social diseases since seriously affect the society in terms of discomfort , pain, care costs, absence from the working places and from the school.The health and operating conditions of the tissues and of the structures of the mouth influence the general physical conditions of the human beings, his aspect, apart from the mastication and speaking capacity, and thus influence the interpersonal relationships. It is thus evident the importance taken by the prevention of the mouth diseases, as well as the keeping thereof in good health conditions.

Among the several causes of diseases of the oral cavity most important both from the point of view of the frequency and from that of the economical incidence are the dental caries and the periodontopathies.

The pathogenesis of the dental caries and of the diseases of the periodontium connected thereto is due to the combined intervention of microbial and dietetical factors.

As a matter of fact the acidophilic bacteria present in the oral cavity, among which mainly the Streptococcus mutans cause the conversion of the food carbohydrates into glucose polymers (levane and mutane) which adhere to the dental surface giving place to the plaque. From the plaque, bearing bacterial colonies, acid groups are developed which are capable in the long run of demineralize the tooth surface. Thus at the basis of the prevention of the caries and of the associated pathology a correct diet and oral hygiene is required in order to prevent the plaque from being formed and to remove it.

The most common prevention methods thus consist in the reduction of the consumption of the plaque generating sugars (mostly saccharose) and in the use of suitable mechanical and chemical means.

However the compliance towards these mechanical means is not always good, especially as regards the childrens who are particularly vulnerable to the cariogenetic processes, and moreover there is not always possible to carry out the operation on the whole dental surface by fully removing the plaque (for example the interdental spaces).

The use of agents chemically active on the plaque to reduce the adhesion thereof to the tooth surface raises use difficulties.

As a matter of fact it is necessary that the active principle is homogeneously distributed and that within the oral cavity concentrations are attained which are active for a sufficiently extended time; the latter condition in particular cannot be readily obtained by the common means since the salivation as caused by the same presence of the vehicle of the active principle tends to cause the concentration thereof to be readily lowered.

According to research work carried out recently with reference to the inhibition of the dental plaque the results have been reported (Bowles W.H., Journal of Dental Research, 68 (Spec.Iss.), 1698,1699, Nov.1989) of tests carried out both in vitro and on volunteers with solutions containing N-acetylcysteine, from which it resulted that the action of this substance does not affect the absorption of the salivary proteins onto the tooth surfaces, but occurs in terms of its capacity of bonding the saliva proteins and/or modifying their affinity towards the sites of bacterial colony growth.

The above reported studies have shown an average 30% reduction of the plaque index in comparison with the controls.

It has been now found and is the subject of the present invention that if N-acetylcysteine is incorporated in a preparation of chewing gum, it is possible to maintain within the mouth amounts of N-acetylcysteine active on the plaque for an extended time and thus achieve much higher reduction levels of the plaque index.

More specifically the main object of the present invention consists of a chewing gum composition which is characterized in that the composition of base gum, per se known but using non cariogenic sweetening substances, is added with a therapeutically effective amount of N-acetylcysteine.

As a matter of fact it has been experimentally found that by using the medicated chewing gum composition according to the present invention the plaque index becomes reduced on the average by 70% with respect to the controls.

An in vitro study has been carried out by using samples of saliva as removed in subsequent times (0, 10, 20 and 40 minutes) from the oral cavity of individuals which masticated the gum medicated with 100 mg of N-acetylcysteine (NAC). The antiplaque activity of the saliva samples containing NAC has been then tested onto a plaque just formed onto artificial preparates (Smith P.K., Ann.Biochem., 150, 76-85, 1985) and it was found that even after 40 minutes the action of reduction of the adhesivity of the plaque to the synthetic preparation was present. During this study it was also observed that the activity was more marked when the pH was slightly alkaline. The activity of the composition of the present invention has been also studied in vivo.

To this end 2 grams of saccharose have been administered to 7 volunteers so as to be dissolved in the mouth and then, by using detecting erythrosin tablets, the plaque index has been calculated (Remfiord S.P.,

J. Periodontal 30, 51-59,1959) before and after the use for 30 minutes of medicated chewing gum containing 100 mg of NAC.

The results are reported in the following table:

% reduction of the plaque index

| Volunteer | gum + NAC |
|---|---|
| AJ 55 M | - 80 |
| ES 52 F | - 79 |
| PG 49 M | - 50 |
| MG 21 F | - 80 |
| AZ 44 F | - 78 |
| CQ 34 F | - 66 |
| MS 17 M | - 59 |
| | ---------- |
| | - 70,3 |

As it can be appreciated the gum medicated with NAC causes the plaque index to be reduced on the average by 70%.

These results show a significatively very effective action of the medicated gum.

According to another test six patients have used for a week chewing gum medicated with 100 mg NAC and five subjects have used for the same period tablets containing 100 mg NAC.

The posology was five chewing gum pills and tablets respectively distributed throughout the day.

By the method above described the plaque has been detected and the plaque index has been evaluated before the treatment and after seven days, two hours after the last administration of the preparation being tested. The results are reported in the following table.

Percent variations of the plaque index

| Volunteers | gum + NAC | Volunteers | tablet + NAC |
|---|---|---|---|
| AG 22 F | -60 | MM 24 F | -30 |
| MM 24 F | -70 | PM 16 M | -37 |
| PM 16 M | -80 | MG 20 F | -40 |
| PB 39 M | -66 | NG 17 F | -45 |
| CQ 34 F | -68 | AG 16 F | -35 |
| GB 36 M | -76 | | |

According to this study the medicated gum has a significatively higher activity than the tablets.

The medicated gum can be prepared by using the techniques known and disclosed in the technical literature whereby a more detailed description is not necessary.

Only for illustrating purpose the qualitative composition of a chewing gum according to the invention is reported hereinafter:

- base gum
- sorbitan
- glycerol
- flavouring essence

- saccharin (sodium salt)
- soy lecithin
- N-acetylcysteine

According to the invention an amount 50 mg NAC per 2.25 g of medicated gum as unit dose is foreseen.

As regards the preparation of the medicated gum the base gum, melted at about 80ºC, is charged in a mixer already containing the other ingredients apart from the active principle.

The latter is separately admixed with part of the sorbitan and then added to the mixer, the mixing being then extended for further 20 minutes.

At the end the unit doses of chewing gum are prepared in the usual way.

The amount of gum in which the active principle is incorporated is determining as regards the release time.As a matter of fact the greater is the gum amount, the longer is the release time. Thus the final composition depends both on the desired oncentration of active principle and on the release time.

The chewing gum can be medicated with other substances having an action like that of NAC such as, as non limiting examples, carboxymethylcysteine and thiopronin. Instead of sorbitan other non cariogenic sugars can be used, such as xylitol and other pentitols or sweetening agents as saccharin, cyclamate, stevioside or aspartame.

The active principles, if longer release time are desired, can be also used in microincapsulated form.

An important feature of the present invention is that the pH is slightly alkaline and is obtained thanks to the choice of the additives of the composition which originate the ideal environnment for the NAC action.

The invention obviously contemplates the possible variations falling within the reach of the skilled technician especially as regards the standard components of the chewing gum and the production technologies , which are too well known in the art.

## Claims

1. Chewing gum composition characterized in that the composition of base gum, per se known but using non cariogenic substances, is added with a therapeutically effective amount of N-acetylcysteine or of a substance having like action.

2. Composition according to claim 1, characterized in that said non cariogenic or sweetening substances are selected among sorbitan, pentitols, saccharin, cyclamate, stevioside and aspartame.

3. Composition according to claim 1, characterized in that said substances having action like that of N-acetylcysteine are carboxymethylcysteine and thiopronin.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,Y | PATENT ABSTRACTS OF JAPAN vol. 11, no. 267 (C-443)(2714) August 28, 1987 & JP-62 070 312 (YAMAZAKI YOJI ) March 31, 1987 * the whole document * _ _ _ | 1,3,1,3 | A 23 G 3/30 A 61 K 9/68 A 61 K 7/22 A 23 G 3/30 + |
| Y,D | JOURNAL OF DENTAL RESEARCH vol. 68, November 1989, pages 1698 - 1699; BOWLES, W.H.: 'N-ACETYLCYSTEINE, A MUCOLYTIC AGENT AS A PLAQUE INHIBITOR ' * the whole document * _ _ _ _ _ | 1,3 | |

TECHNICAL FIELDS
SEARCHED (Int. Cl.5)

A 23 G
A 61 K

The present search report has been drawn up for all claims

| Place of search | Date of completion of search | Examiner |
|---|---|---|
| The Hague | 10 October 91 | LEPRETRE F.G.M.J. |

CATEGORY OF CITED DOCUMENTS
X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons
 
& : member of the same patent family, corresponding document